(19) Europäisches Patentamt
European Patent Office
Office européen des brevets

(11) **EP 2 298 294 A1**

(12) **EUROPEAN PATENT APPLICATION**
published in accordance with Art. 153(4) EPC

(43) Date of publication:
**23.03.2011 Bulletin 2011/12**

(21) Application number: **09766595.4**

(22) Date of filing: **12.06.2009**

(51) Int Cl.:
*A61K 31/245* (2006.01)   *A61K 9/06* (2006.01)
*A61K 9/08* (2006.01)   *A61K 9/10* (2006.01)
*A61K 9/12* (2006.01)   *A61K 9/14* (2006.01)
*A61K 9/70* (2006.01)   *A61P 17/00* (2006.01)
*A61P 29/00* (2006.01)

(86) International application number:
**PCT/JP2009/060767**

(87) International publication number:
**WO 2009/154147 (23.12.2009 Gazette 2009/52)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **16.06.2008 JP 2008156712**
**11.05.2009 JP 2009114164**

(71) Applicant: **TEIKOKU SEIYAKU CO., LTD.**
**Higashikagawa-shi, Kagawa 769-2695 (JP)**

(72) Inventors:
• **INOO, Katsuyuki**
**Higashikagawa-shi**
**Kagawa 769-2695 (JP)**

• **KAWADA, Mitsuhiro**
**Higashikagawa-shi**
**Kagawa 769-2695 (JP)**
• **MORI, Kenjiro**
**Higashikagawa-shi**
**Kagawa 769-2695 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **OXYBUPROCAINE-CONTAINING ANALGESIC/ANTIPRURITIC PREPARATION FOR EXTERNAL APPLICATION**

(57) An analgesic/antipruritic external preparation that includes a local anesthetic, has fewer side effects, and has an excellent therapeutic effect on pain and itching of the skin is provided. The analgesic/antipruritic external preparation includes oxybuprocaine or a pharmaceutically acceptable salt thereof as an active ingredient, and the oxybuprocaine or a pharmaceutically acceptable salt thereof is contained in an amount of 0.1 to 60 wt%, more preferably 1 to 40 wt%, and most preferably 5 to 30 wt%. The analgesic/antipruritic external preparation has a dosage form as an external preparation wherein the dosage form is an ointment, a solution, a suspension, an emulsion, a lotion, a cataplasm, a tape, an aerosol, or a powder for external use.

EP 2 298 294 A1

**Description**

TECHNICAL FIELD

The present invention relates to an external preparation that includes oxybuprocaine or a pharmaceutically acceptable salt thereof as an active ingredient and has a highly therapeutic effect on itching and pain in the skin, and a method for treating pain and itching of the skin using the external preparation.

BACKGROUND ART

Development of external preparations that include various local anesthetics such as lidocaine has been conventionally under investigation, and external preparations that have excellent analgesic action or local anesthetic action have been reported (for example, Patent Documents 1 to 3).
Such external preparations are classified as external preparations that are applied to alleviate persistent pain such as herpes zoster or postherpetic neuralgia (Patent Documents 1 and 3) or external preparations that alleviate pain at the time of a puncture (Patent Document 2), and are not classified as external preparations for pain and itching of the skin.
Although formulations containing local anesthetics that utilize antipruritic action of the local anesthetics have been proposed for pain and itching of the skin, there are currently few reports on external preparations that have both a high level of medicinal effect and safety.
For example, Patent Document 4 proposes an analgesic/antipruritic external preparation that includes a local anesthetic and vitamin E, and squalane to reduce the side effects of the local anesthetic; however, the external preparation includes a relatively large amount of vitamin E to reduce the side effects of the local anesthetic, and its safety on the skin is not satisfactory.
Therefore, there is currently a need for development of safe and more effective analgesic/antipruritic external preparations for pain and itching of the skin.
Patent Document 1: Japanese Patent Application Laid-Open No. Hei 4-305523

Patent Document 2: Japanese Patent Application Laid-Open No. Hei 6-145051

Patent Document 3: Japanese Patent Application Laid-Open No. Hei 10-147521

Patent Document 4: Japanese Patent Application Laid-Open No. Hei 10-316564

DISCLOSURE OF THE INVENTION

PROBLEMS TO BE SOLVED BY THE INVENTION

The present invention solves the above-mentioned problems. It is an object of the present invention to provide an analgesic/ antipruritic external preparation that includes a local anesthetic, has fewer side effects, and has an excellent therapeutic effect on pain and itching of the skin.
The present inventors conducted a diligent examination to solve the above-mentioned problems. As a result of a comparative examination of the analgesic effects and antipruritic effects of various local anesthetics, the present inventors found that oxybuprocaine has a very high level of analgesic and antipruritic action among the local anesthetics. The present inventors prepared an external preparation that included oxybuprocaine or pharmaceutically acceptable salts thereof as an active ingredient and confirmed that a highly therapeutic effect on pain and itching of the skin was produced by applying the external preparation to the skin.
Specifically, the present inventors prepared an analgesic/antipruritic external preparation that included a local anesthetic, oxybuprocaine, applied this formulation to an affected skin area with pain or itching, and found that the formulation had a very high level of analgesic/antipruritic effect, thereby accomplishing the present invention.

MEANS FOR SOLVING THE PROBLEMS

Accordingly, a basic embodiment of the present invention is an analgesic/antipruritic external preparation that includes oxybuprocaine or a pharmaceutically acceptable salt thereof as an active ingredient.
Preferably, the above-mentioned analgesic/antipruritic external preparation includes oxybuprocaine or a pharmaceutically acceptable salt thereof at a content of 0.1 to 60 wt% based on the total weight of the preparation. More preferably, the above-mentioned analgesic/antipruritic external preparation includes oxybuprocaine or a pharmaceutically acceptable salt thereof at a content of 1 to 40 wt% based on the total weight of the preparation, and most preferably, includes oxybuprocaine or a pharmaceutically acceptable salt thereof at a content of 5 to 30 wt% based on the total weight of the preparation.

Additionally, the present invention is specifically the above-mentioned analgesic/antipruritic external preparation whose dosage form as an external preparation is an ointment, a solution, a suspension, an emulsion, a lotion, a cataplasm, a tape, an aerosol, or a powder for external use.

One of the most preferable embodiments of the present invention is an analgesic/antipruritic external preparation in the form of a tape that includes oxybuprocaine or pharmaceutically acceptable salts thereof at a content of 5 to 30 wt% in an adhesive base.

Furthermore, the present invention is, in another embodiment, the above-mentioned analgesic/antipruritic external preparation that includes oxybuprocaine or a pharmaceutically acceptable salt thereof, which is used for treatment of pain and itching of the skin, and moreover, a method of using the above-mentioned analgesic/ antipruritic external preparation that includes oxybuprocaine for treatment of pain and itching of the skin.

EFFECTS OF THE INVENTION

The analgesic/antipruritic external preparation of the present invention includes oxybuprocaine as an active ingredient, thereby producing an excellent therapeutic effect on pain and itching of the skin. Specifically, the analgesic/ antipruritic external preparation of the present invention is very effective against atopic dermatitis, eczema, contact dermatitis, seborrheic dermatitis, urticaria, strophulus infantum, insect sting, cutaneous pruritus; itching associated with metabolic diseases such as uremia and chronic renal failure, endocrine diseases such as diabetes, and the like, and diseases associated with itching, for example itching associated with skin wounds such as cut wounds, postoperative wounds, and burn wounds; chronic pain such as chronic rheumatoid arthritis, osteoarthritis, and lumbago; inflammatory diseases such as periarthritis scapulohumeralis and tendovaginitis; diseases associated with pain such as pain resulting from a surgery, a trauma, and the like; or neuropathic pain.

Thus, the present invention provides external preparations in various dosage forms, which have a sufficient therapeutic effect on various types of pain and itching of the skin, have very few side effects, and are useful for treatment of pain and itching. These external preparations have great medical value.

BEST MODE FOR CARRYING OUT THE INVENTION

Oxybuprocaine included as an active ingredient in the external preparation provided by the present invention is a drug that was developed as a local anesthetic, has surface anesthesia action, infiltration anesthesia action, and conduction anesthesia action, and is mainly used for surface anesthesia in the ophthalmologic field.

As described above, the basic embodiment of the present invention is an analgesic/antipruritic external preparation that includes such oxybuprocaine or pharmaceutically acceptable salts thereof as an active ingredient.

Although the content varies depending on the dosage form as the external preparation and is not necessarily limited, it may be the amount sufficient to exert the desired analgesic/antipruritic effect. Specifically, the content may be 0.1 to 60 wt%, preferably 1 to 40 wt%, and more preferably 5 to 30 wt% based on the total weight of the formulation.

Note that the above-mentioned total weight of the formulation refers to the total weight of the paste when the inventive external preparation is a cataplasm, and the total weight of the adhesive when the inventive external preparation is a tape.

When the content is more than 60 wt%, retention of the physical properties as an external preparation becomes difficult. Content exceeding this weight may not enhance the effect. On the other hand, when the content is less than 0.1 wt%, the analgesic/antipruritic action of oxybuprocaine may not be exerted satisfactorily and the desired analgesic/ antipruritic effect may not be obtained.

The external preparation provided by the present invention is not particularly limited as long as its dosage form allows direct administration of an active ingredient to the local surface of the skin. For example, formulations such as an ointment, a solution (a suspension, an emulsion, a lotion, and the like), a cataplasm, a tape, an aerosol, and a powder for external use may be prepared and used.

In preparing these formulations, various ingredients that are used to prepare ordinary external preparations may be selected and used as appropriate, in addition to oxybuprocaine as an active ingredient.

Such ingredients, in the case of an ointment, a cream, a gel, and a lotion, may include bases such as white petrolatum, yellow petrolatum, lanolin, white beeswax, cetanol, stearyl alcohol, stearic acid, hydrogenated oil, hydrocarbon gel, polyethylene glycol, liquid paraffin, and squalane; solvents and solubilizers such as oleic acid, isopropyl myristate, glyceryl triisooctanoate, crotamiton, diethyl sebacate, diisopropyl adipate, hexyl laurate, fatty acids, fatty acid esters, aliphatic alcohols, and vegetable oil; antioxidants such as tocopherol derivatives, L-ascorbic acid, dibutylhydroxytoluene, and butylated hydroxyanisole; antiseptics such as p-hydroxybenzoic acid esters; humectants such as glycerin, propylene glycol, and sodium hyaluronate; surfactants such as polyoxyethylene derivatives, glycerine fatty acid esters, sucrose fatty acid esters, sorbitan fatty acid esters, propylene glycol fatty acid esters, and lecithin; thickeners such as carboxyvinyl polymers, xanthan gum, carboxymethylcellulose, carboxymethylcellulose sodium salts, hydroxypropylcellulose, and hydroxypropylmethylcellulose; and the like.

Additionally, a stabilizer, a preservative, an absorbefacient, a pH adjustor, and other suitable additives may be blended if desired.

In the case of a cataplasm, such ingredients may include tackifiers such as polyacrylic acid and polyacrylate copolymers; cross-linking agents such as aluminum sulfate, aluminum potassium sulfate, aluminum chloride, magnesium aluminometasilicate, and dihydroxyaluminum acetate; thickeners such as sodium polyacrylate, polyvinyl alcohol, polyvinylpyrrolidone, gelatin, sodium alginate, carboxymethylcellulose, carboxymethylcellulose sodium salts, hydroxypropylcellulose, and hydroxypropylmethylcellulose; polyalcohols such as glycerin, polyethylene glycol (macrogol), propylene glycol, and 1,3-butanediol; surfactants such as polyoxyethylene derivatives; perfumes such as 1-menthol; antiseptics such as p-hydroxybenzoic acid esters; purified water; and the like.

Additionally, a stabilizer, a preservative, an absorbefacient, a pH adjustor, and other suitable additives may be blended if desired.

In the case of a tape, an adhesive such as styrene-isoprene-styrene block copolymers (SIS block copolymers) and acrylic resin; a tackifier resin such as alicyclic saturated hydrocarbon resin, rosin-based resin, and terpene-based resin; a softener such as liquid rubber and liquid paraffin; an antioxidant such as dibutylhydroxytoluene; a polyalcohol such as propylene glycol; an absorbefacient such as oleic acid; a surfactant such as polyoxyethylene derivatives; and other suitable additives may be blended.

Furthermore, a water-containing tape may be formulated by adding polymers such as sodium polyacrylate and polyvinyl alcohol, which can retain water, and a small amount of purified water.

In this case, additionally, a stabilizer, a preservative, an absorbefacient, a pH adjustor, and other suitable additives may also be blended if desired.

In the case of an aerosol, a base such as white petrolatum, yellow petrolatum, lanolin, white beeswax, cetanol, stearyl alcohol, stearic acid, hydrogenated oil, hydrocarbon gel, polyethylene glycol, liquid paraffin, and squalane; a solvent and a solubilizer such as oleic acid, isopropyl myristate, diisopropyl adipate, isopropyl sebacate, glyceryl triisooctanoate, crotamiton, diethyl sebacate, hexyl laurate, fatty acids, fatty acid esters, aliphatic alcohols, and vegetable oil; an antioxidant such as tocopherol derivatives, L-ascorbic acid, dibutylhydroxytoluene, and butylated hydroxyanisole; antiseptics such as p-hydroxybenzoic acid esters; humectants such as glycerin, propylene glycol, and sodium hyaluronate; a surfactant such as polyoxyethylene derivatives, glycerine fatty acid esters, sucrose fatty acid esters, sorbitan fatty acid esters, propylene glycol fatty acid esters, and lecithin; a thickener such as carboxyvinyl polymers, xanthan gum, carboxymethylcellulose, carboxymethylcellulose sodium salts, hydroxypropylcellulose, and hydroxypropylmethylcellulose, which are used for preparation of an ointment, a cream, a gel, a suspension, an emulsion, a solution, a lotion, and the like; and moreover, various stabilizers, buffering agents, flavoring agents, suspending agents, emulsifiers, perfuming agents, preservatives, solubilizers, and other suitable additives may be blended.

In the case of a powder for external use, excipients such as potato starch, rice starch, cornstarch, talc, and zinc oxide or other suitable additives may be blended.

In this case, additionally, various stabilizers, preservatives, absorbefacients, and other suitable additives may also be blended if desired.

The procedure of preparing the external preparation provided by the present invention is not particularly limited. The external preparation of the present invention is produced using a method of producing an ordinary external preparation such as by thoroughly kneading the ingredients and base ingredients as needed, wherein the method depends on the desired dosage form.

In the preparation of a cataplasm and a tape, they may be prepared by spreading a kneaded mixture on a release liner, drying it, furthermore laminating it to a flexible backing layer, and cutting it to a desired size.

For example, when the external preparation provided by the present invention is an ointment, a solution (a suspension, an emulsion, a lotion, and the like), an aerosol, or a powder for external use, it is used by an ordinary method of use; for example, it is directly applied, for example by application to an affected skin area, or it is applied by using a backing layer such as a cloth coated or impregnated with the preparation.

A cataplasm or a tape is used by a method of directly applying these formulations to an affected skin area.

The external preparations provided by the present invention have different durations of application depending on their dosage forms. For example, in the case of patches such as a tape and a cataplasm, the analgesic/antipruritic effect appeared approximately 15 minutes to 1 hour after application to the skin, and the effect was sustained even after peeling off the patch.

Similarly, in the case of an ointment and solutions such as a suspension, an emulsion, and a lotion, the analgesic/antipruritic effect appeared approximately 15 minutes to 1 hour after application, when they were applied such as by application onto the skin surface.

[Examples]

Hereinbelow, the oxybuprocaine-containing external preparation provided by the present invention will be described with reference to examples and test examples, but the present invention is not intended to be limited to these examples in any way.

Examples 1 to 3:

Based on the formulation shown in Table 1 below, a styreneisoprene-styrene block copolymer (SIS block copolymer), alicyclic saturated hydrocarbon resin, a hydrogenated rosin glycerol ester, liquid paraffin, polybutene, an antioxidant, and the like were added, and mixed and dissolved using toluene. Oxybuprocaine was added to the mixture and mixed, and the mixture obtained by thorough kneading was spread on a release liner, and then toluene was evaporated. The mixture spread on the release liner was laminated to a flexible backing layer and cut to a desired size to obtain a tape.

Comparative Examples 1 to 2:

Based on the formulation shown in Table 1, a styrene-isoprene-styrene block copolymer (SIS block copolymer), alicyclic saturated hydrocarbon resin, a hydrogenated rosin glycerol ester, liquid paraffin, polybutene, an antioxidant, and the like were added, and mixed and dissolved using toluene. The mixture was spread on a release liner, and then toluene was evaporated. The mixture spread on the release liner was laminated to a flexible backing layer and cut to a desired size to obtain a tape.

[Table 1]

| Ingredients | Examples | | | Comparative Examples | |
|---|---|---|---|---|---|
| | 1 | 2 | 3 | 1 | 2 |
| Oxybuprocaine | 5 | 15 | 30 | - | - |
| SIS block copolymer | 20 | 30 | 39 | 20 | 30 |
| Alicyclic saturated hydrocarbon resin | 30 | - | - | 30 | - |
| Hydrogenated rosin glycerol ester | - | 40 | 30 | - | 40 |
| Polybutene | 10 | - | - | 10 | - |
| Liquid paraffin | 34 | 14 | - | 39 | 29 |
| Dibutylhydroxytoluene | 1 | 1 | 1 | 1 | 1 |
| Unit: part by weight | | | | | |

Examples 4 to 5:

Oxybuprocaine was dissolved in propylene glycol based on the formulation shown in Table 2. The solution was kneaded with the other ingredients shown in Table 2 until the mixture became homogeneous, thereby obtaining a drug-containing base. The drug-containing base was spread onto a nonwoven fabric and a polypropylene liner was applied thereto, and the resultant material was cut to a desired size to obtain a cataplasm.

[Table 2]

| Ingredients | Examples | |
|---|---|---|
| | 4 | 5 |
| Oxybuprocaine | 1 | 5 |
| Propylene glycol | 5 | 10 |
| Castor oil | 0.5 | 1 |

(continued)

| Ingredients | Examples | |
|---|---|---|
| | 4 | 5 |
| Glycerin | 15 | 15 |
| Polyacrylic acid | 4 | 4 |
| Partially neutralized polyacrylic acid | 5 | 5 |
| Carboxymethylcellulose sodium | 4 | 4 |
| Aluminum hydroxide | 0.5 | 0.5 |
| Magnesium aluminometasilicate | 0.03 | 0.03 |
| Tartaric acid | 0.5 | 0.5 |
| Edetate disodium | 0.04 | 0.04 |
| Purified water | Balance | Balance |
| Unit: part by weight | | |

Test Example 1: A pharmacological test of antipruritic action using mice

Oxybuprocaine in saline was administered transdermally to a male ddy strain mouse in the dorsal neck area at a dose of 10 mg/kg. Then, Compound 48/80 was administered subcutaneously at 100 $\mu$g/body to induce itching. Scratching behaviors in the mouse were monitored for 30 minutes after induction and the number of times scratching occurred was measured.

The group that received only saline (a control group) and the group that intraperitoneally received an antihistamine, cyproheptadine at 1 mg/kg (a positive control group) served as control groups.

The results are shown in Table 3 below.

[Table 3]

| Test groups | n | Number of times scratching occurred (mean $\pm$ standard error) |
|---|---|---|
| Control group (Group received saline) | 6 | 110.0 $\pm$ 19.1 |
| Positive control group (Group received cyproheptadine) | 6 | 76.7 $\pm$ 21.3 |
| Group received oxybuprocaine | 6 | 45.8 $\pm$ 18.9* |
| *: $p < 0.05$ (relative to a control group) | | |

As is found from the results shown in the table, subcutaneous administration of the oxybuprocaine-containing aqueous solution of the present invention suppressed scratching behaviors strongly compared to the control group (the group that received saline), and suppressed scratching behaviors more strongly than the group that received an antihistamine, cyproheptadine (the positive control group). Thus, the subcutaneous administration provided an excellent antipruritic effect.

Test Example 2: A pharmacological test of analgesic action using rats

The pain threshold of a male Wistar strain rat (4-week old) was measured using Analgesy Meter at its right footpad and subsequently a test substance was applied to the right footpad. Tapes from Example 2 and Comparative Example 2 were used as test substances.

The test substances were removed 4 hours after application and 0.1 mL of suspension of brewer's yeast was injected subcutaneously into the right foot. The pain threshold at the right footpad was measured 1 hour, 2 hours, and 3 hours after the injection of the suspension of brewer's yeast, and the percentage of the pain threshold (pain threshold ratio) was calculated relative to the pain threshold measured before the application of the test substances. The percentage of the pain threshold (pain threshold ratio) was calculated following the formula below.

```
    Pain threshold ratio = (pain threshold after treatment with
brewer's yeast/pain threshold before treatment with brewer's yeast)
x 100
```

An untreated group to which no test substances were applied served as a control group. The results are shown in Table 4 below.

[Table 4]

| Test groups (test substances) | n | Pain threshold ratio (mean ± standard error) | | |
| --- | --- | --- | --- | --- |
| | | Injection of brewer's yeast | | |
| | | 1 hr. later | 2 hrs. later | 3 hrs. later |
| Control group (untreated group) | 8 | 0.47 ± 0.08 | 0.52 ± 0.05 | 0.48 ± 0.06 |
| Example 2 | 8 | 0.68 ± 0.06 (43.5) | 0.63 ± 0.04 (20.1) | 0.67 ± 0.06 (40.0) |
| Comparative examples 2 | 8 | 0.60 ± 0.06 (26.6) | 0.50 ± 0.04 (-4.3) | 0.61 ± 0.04 (25.9) |

Numbers in brackets denote the percentage of elevation relative to the control group.

As is found the results shown in the table, the tape of Example 2 that included oxybuprocaine showed a greater analgesic effect compared to the tape base of Comparative Example 2 that included no oxybuprocaine.

Test Example 3:

To study a pharmacological effect of the analgesic/antipruritic external preparations of the present invention, a patch test was performed using test subjects (10 male volunteers).

[Test method]

The tapes from Examples 1 and 3, which were the tapes of the present invention, and the tapes from Comparative Examples 1 and 2 were applied to the inside parts of upper arms of the 10 test subjects (male). The tapes were peeled off six hours after application. The parts where the tapes were peeled off were stimulated with an injection needle (21-gauge) (a puncture), and the analgesic effect at that time was evaluated by a sensory test.

Evaluation criteria of stimulation are as follows:

-: no pain was sensed
+: a weak pain was sensed
++: a strong pain was sensed

[Results]

The results are shown in Table 5 below.

[Table 5]

| Evaluation criteria | Numbers of subjects | | | |
|---|---|---|---|---|
| | Example | | Comparative example | |
| | 1 | 3 | 1 | 2 |
| - | 7 | 10 | 0 | 0 |
| + | 3 | 0 | 0 | 0 |
| ++ | 0 | 0 | 10 | 10 |

As is found from the results shown in Table 5, while the application of the inventive tapes from Examples 1 and 3 that included oxybuprocaine produced a highly analgesic effect, the application of the tapes from Comparative Examples 1 and 2 that included no oxybuprocaine produced no analgesic effect.

INDUSTRIAL APPLICABILITY

As described above, the present invention provides external preparations in various dosage forms, which have a sufficient therapeutic effect on various types of pain and itching of the skin, have very few side effects, and are useful for treatment of pain and itching.
Therefore, the present invention has great medical value, since there have been no external preparations to date that are safe and have highly effective analgesic/antipruritic action.

**Claims**

1.  An analgesic/antipruritic external preparation, comprising oxybuprocaine or a pharmaceutically acceptable salt thereof as an active ingredient.

2.  The analgesic/antipruritic external preparation according to claim 1, wherein a content of oxybuprocaine or the pharmaceutically acceptable salt thereof is 0.1 to 60 wt% based on a total weight of the preparation.

3.  The analgesic/antipruritic external preparation according to claim 1, wherein a content of oxybuprocaine or the pharmaceutically acceptable salt thereof is 1 to 40 wt% based on a total weight of the preparation.

4.  The analgesic/antipruritic external preparation according to claim 1, wherein a content of oxybuprocaine or the pharmaceutically acceptable salt thereof is 5 to 30 wt% based on a total weight of the preparation.

5.  The analgesic/antipruritic external preparation according to any of claims 1 to 4, wherein a dosage form is an ointment, a solution, a suspension, an emulsion, a lotion, a cataplasm, a tape, an aerosol, or a powder for external use.

6.  The analgesic/antipruritic external preparation according to claim 1, being in the form of a tape that includes oxybuprocaine or the pharmaceutically acceptable salt thereof at a content of 5 to 30 wt% in an adhesive base.

7.  An analgesic/antipruritic external preparation that includes oxybuprocaine or a pharmaceutically acceptable salt thereof according to any of claims 1 to 6, which is used for treatment of pain and itching of the skin.

8.  A method of using the analgesic/antipruritic external preparation that includes oxybuprocaine or a pharmaceutically acceptable salt thereof according to any of claims 1 to 6, for treatment of pain and itching of the skin.

### INTERNATIONAL SEARCH REPORT

| | International application No. |
|---|---|
| | PCT/JP2009/060767 |

A. CLASSIFICATION OF SUBJECT MATTER
*A61K31/245*(2006.01)i, *A61K9/06*(2006.01)i, *A61K9/08*(2006.01)i, *A61K9/10* (2006.01)i, *A61K9/12*(2006.01)i, *A61K9/14*(2006.01)i, *A61K9/70*(2006.01)i, *A61P17/00*(2006.01)i, *A61P29/00*(2006.01)i
According to International Patent Classification (IPC) or to both national classification and IPC

B. FIELDS SEARCHED

Minimum documentation searched (classification system followed by classification symbols)
A61K31/245, A61K9/06, A61K9/08, A61K9/10, A61K9/12, A61K9/14, A61K9/70, A61P17/00, A61P29/00

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)
BIOSIS(STN), CAplus(STN), EMBASE(STN), MEDLINE(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-001441 A  (Amato Pharmaceutical Products Ltd.),<br>06 January, 1998 (06.01.98),<br>Particularly, Claims; Par. Nos. [0013], [0016]<br>(Family: none) | 1-5,7<br>5-7 |
| X<br>Y | WO 2005/007140 A1  (KIM J H),<br>27 January, 2005 (27.01.05),<br>Particularly, Claims; page 4, lines 14 to 21;<br>page 15, lines 7 to 12; page 7, line 8 to<br>page 9, line 9<br>& KR 2005009502 A | 1-5,7<br>5-7 |
| X<br>Y | Takahiro AKAISHI et al., "Heiken Hanno o Shihyo to shita Kato Kakumaku Chikaku Hyokakei no Kakuritsu", Folia Ophthalmogica Japonica, 2001, Vol.52, No.5, pages 377 to 382 | 1-5<br>5,6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>24 June, 2009 (24.06.09) | Date of mailing of the international search report<br>07 July, 2009 (07.07.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/060767 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2000-178186 A  (Yutoku Pharmaceutical Ind. Co., Ltd., Maruho Kabushiki Kaisha), 27 June, 2000 (27.06.00), Particularly, Claims; examples 1 to 3 & WO 2000/019986 A1     & EP 1125578 A1 & US 6953590 B1 | 5-7 |
| Y | JP 2000-319168 A  (Yutoku Pharmaceutical Ind. Co., Ltd.), 21 November, 2000 (21.11.00), Particularly, Claims; examples 1 to 4 (Family: none) | 5-7 |

Form PCT/ISA/210 (continuation of second sheet) (April 2007)

| **INTERNATIONAL SEARCH REPORT** | International application No. |
|---|---|
| | PCT/JP2009/060767 |

---

**Box No. II    Observations where certain claims were found unsearchable (Continuation of item 2 of first sheet)**

This international search report has not been established in respect of certain claims under Article 17(2)(a) for the following reasons:

1. [X] Claims Nos.: 8
    because they relate to subject matter not required to be searched by this Authority, namely:
    Claim 8 pertains to methods for treatment of the human body by therapy and thus relates to a subject matter which this International Searching Authority is not required, under the provisions of Rule 39.1(iv) of the Regulations under the PCT, to search.

2. [ ] Claims Nos.:
    because they relate to parts of the international application that do not comply with the prescribed requirements to such an extent that no meaningful international search can be carried out, specifically:

3. [ ] Claims Nos.:
    because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
    The inventions of claims 1-5 and the invention of claim 7 are not novel, as disclosed in Documents 1-3 and Documents 1 and 2, respectively. Therefore, the inventions have no special technical feature.

Document 1: JP 10-001441 A (Amato Pharmaceutical Products Ltd.) 06 January, 1998 (06.01.98)
Document 2: WO 2005/007140 A1 (KIM J H) 27 January, 2005 (27.01.05)
Document 3: Takahiro AKAISHI et al., "Heiken Hanno o Shihyo to shita Kato Kakumaku Chikaku Hyokakei no Kakuritsu", Folia Ophthalmogica Japonica, 2001, Vol.52, No.5, pages 377 to 382

1. [ ] As all required additional search fees were timely paid by the applicant, this international search report covers all searchable claims.

2. [X] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI4305523 B
- JP HEI6145051 B
- JP HEI10147521 B
- JP HEI10316564 B